# EUROPEAN PATENT SPECIFICATION

(11) **EP 0 969 854 B1**
(45) Date of publication and mention of the grant of the patent: **01.02.2006**
(21) Application number: 97936092.2
(22) Date of filing: 16.07.1997
(51) Int. Cl.: A61K 38/02, A61K 38/03, A61K 38/04, A61K 38/07, A61K 38/08, A61K 38/10, A61K 38/16, C07K 2/00

(54) **INHIBITION OF SIGNAL TRANSDUCTION BY THE BINDING OF NON-PHOSPHORYLATED COMPOSITIONS**
UNTERDRÜCKUNG DER SIGNALWEITERLEITUNG DURCH DIE BINDUNG NICHT-PHOSPHORYLIERTER ZUSAMMENSETZUNGEN
INHIBITION DE LA TRANSDUCTION DE SIGNAL PAR LA FIXATION DE COMPOSITIONS NON PHOSPHORYLEES

(30) Priority: 16.07.1996 US 21858 P
(43) Date of publication of application: 12.01.2000
(73) Proprietor: GEORGETOWN UNIVERSITY, Washington, D.C. 20057 (US); THE UNIVERSITY OF VERMONT AND STATE AGRICULTURAL COLLEGE, Burlington, VT 05405-0160 (US)
(72) Inventor: KING, Carol Richter, Washington, DC 20010 (US); SASTRY, Lakshmi, Bethesda, MD 20814 (US); KRAG, David, Shelburne, VT 05482 (US); OLIGINO, Lyn, Richmond, VT 05477 (US)
(74) Representative: Guerre, Dominique
(86) International application number: PCT/US1997/012501
(87) International publication number: WO 1998/002176

(56) References cited:
- WO-A-96/23813
- YE BIN ET AL: "L-O-(2-malonyl)tyrosine: A new phosphotyrosyl mimetic for the preparation of Src homology 2 domain inhibitory peptides." JOURNAL OF MEDICINAL CHEMISTRY, vol. 38, no. 21, 1995, pages 4270-4275, XP002215445 ISSN: 0022-2623

### SEQUENCE LISTING

(1) GENERAL INFORMATION:
   (i) APPLICANT: KING, C. RICHTER SASTRY, LAKSHMI KRAG, DAVID OLIGINO, LYN
   (ii) TITLE OF INVENTION: INHIBITION OF SIGNAL TRANSDUCTION BY THE BINDING OF NON-PHOSPHORYLATED COMPOSITIONS
   (iii) NUMBER OF SEQUENCES: 14
   (iv) CORRESPONDENCE ADDRESS:
      (A) ADDRESSEE: OLIFF & BERRIDGE, PLC
      (B) STREET: P.O. BOX 19928
      (C) CITY: ALEXANDRIA
      (D) STATE: VA
      (E) COUNTRY: USA
      (F) ZIP: 22320
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER: PCT/US97/12501
      (B) FILING DATE: 16-JUL-1997
      (C) CLASSIFICATION:
   (viii) ATTORNEY/AGENT INFORMATION:
      (A) NAME: BERRIDGE, WILLIAM P
      (B) REGISTRATION NUMBER: 30,024
      (C) REFERENCE/DOCKET NUMBER: WPB 38338(PC)
   (ix) TELECOMMUNICATION INFORMATION:
      (A) TELEPHONE: (703)-836-6400
      (B) TELEFAX: (703)-836-2787
(2) INFORMATION FOR SEQ ID NO:1:
   (i) SEQUENCE CHARACTERISTICS:
      (A) LENGTH: 9 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 5
      (D) OTHER INFORMATION: /note= "PHOSPHOTYROSINE"
   (xi) SEQUENCE DESCRIPTION: SEQ ID NO:1:
   Asp Asp Pro Ser Xaa Val Asn Val Gln
   1               5
(2) INFORMATION FOR SEQ ID NO:2:
   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (xi) SEQUENCE DESCRIPTION: SEQ ID NO:2:
   Cys Glu Leu Tyr Glu Asn Val Gly Met Tyr Cys
   1               5                   10
(2) INFORMATION FOR SEQ ID NO:3:
   (i) SEQUENCE CHARACTERISTICS:
      (A) LENGTH: 5 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 1
      (D) OTHER INFORMATION: /note= "ACETYLATED PHOSPHOTYROSINE"
   (xi) SEQUENCE DESCRIPTION: SEQ ID NO:3:
   Xaa Glu Glu Ile Glu
   1               5
(2) INFORMATION FOR SEQ ID NO:4:
   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (xi) SEQUENCE DESCRIPTION: SEQ ID NO:4:
   Ser Glu Leu Tyr Glu Asn Val Gly Met Tyr Ser
   1               5                   10
      INFORMATION FOR SEQ ID NO:5:

   (i) SEQUENCE CHARACTERISTICS:
      (A) LENGTH: 9 amino acids
      (B) TYPE: amino acid
      (C) STRANDEDNESS:
      (D) TOPOLOGY: not relevant
   (ii) MOLECULE TYPE: peptide
   (xi) SEQUENCE DESCRIPTION: SEQ ID NO:5:
   Asp Asp Pro Ser Tyr Val Asn Val Gln
   1               5
      INFORMATION FOR SEQ ID NO:6:

   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (xi) SEQUENCE DESCRIPTION: SEQ ID NO:6:
   Cys Glu Leu Ala Glu Asn Val Gly Met Tyr Cys
   1               5                   10
(2) INFORMATION FOR SEQ ID NO:7:
   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (xi) SEQUENCE DESCRIPTION: SEQ ID NO:7:
   Cys Glu Leu Tyr Glu Ala Val Gly Met Tyr Cys
   1               5                   10
      INFORMATION FOR SEQ ID NO:8:

   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 6
      (D) OTHER INFORMATION: /note= "PHOSPHOTYROSINE"
   (xi) SEQUENCE DESCRIPTION: SEQ ID NO:8:
   Leu Pro Val Pro Glu Xaa Ile Asn Gln Ser Val
   1               5                   10
(2) INFORMATION FOR SEQ ID NO:9:
   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 6
      (D) OTHER INFORMATION: /note= "PHOSPHOTYROSINE"
   (xi) SEQUENCE DESCRIPTION: SEQ ID NO:9: Val Gly Asn Pro Glu Xaa Leu Asn Thr Val Gln 1 5 10
(2) INFORMATION FOR SEQ ID NO:10:
   (i) SEQUENCE CHARACTERISTICS:
      (A) LENGTH: 10 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 5
      (D) OTHER INFORMATION: /note= "PHOSPHOTYROSINE"
   (xi) SEQUENCE DESCRIPTION: SEQ ID NO:10:
   Gln Asn Pro Glu Xaa Leu Asn Thr Val Gln
   1               5                   10
(2) INFORMATION FOR SEQ ID NO:11:
   (i) SEQUENCE CHARACTERISTICS:
      (A) LENGTH: 11 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 6
      (D) OTHER INFORMATION: /note= "PHOSPHOTYROSINE"
   (xi) SEQUENCE DESCRIPTION: SEQ ID NO:11:
   Phe Asp Asp Pro Ser Xaa Val Asn Tyr Gln Asn
   1 5 10
(2) INFORMATION FOR SEQ ID NO:12:
   (i) SEQUENCE CHARACTERISTICS:
      (A) LENGTH: 4 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 2
      (D) OTHER INFORMATION: /note= "PHOSPHOTYROSINE"
   (xi) SEQUENCE DESCRIPTION: SEQ ID NO:12:
   Pro Xaa Leu Asn
   1
(2) INFORMATION FOR SEQ ID NO:13:
   (i) SEQUENCE CHARACTERISTICS:
      (A) LENGTH: 3 amino acids
      (B) TYPE: amino acid
      (C) STRANDEDNESS:
      (D) TOPOLOGY: not relevant
   (ii) MOLECULE TYPE: peptide
   (ix) FEATURE:
      (A) NAME/KEY: Modified-site
      (B) LOCATION: 1
      (D) OTHER INFORMATION: /note= "PHOSPHOTYROSINE"
   (ix) FEATURE:
      (A) NAME/KEY: Peptide
      (B) LOCATION: 2
      (D) OTHER INFORMATION: /note= "UNKNOWN AMINO ACID"
   (xi) SEQUENCE DESCRIPTION: SEQ ID NO:13:
   Xaa Xaa Asn
   1
(2) INFORMATION FOR SEQ ID NO:14:
   (i) SEQUENCE CHARACTERISTICS:
      (A) LENGTH: 9 amino acids
      (B) TYPE: amino acid
      (C) STRANDEDNESS:
      (D) TOPOLOGY: not relevant
   (ii) MOLECULE TYPE: peptide
   (xi) SEQUENCE DESCRIPTION: SEQ ID NO:14:
   Glu Leu Tyr Glu Asn Val Gly Met Tyr
   1               5

## Description

### BACKGROUND OF THE INVENTION

The present invention is directed to a new class of SH2 domain binding peptides. The tyrosines of these peptides are not modified by phosphates or other similarly charged groups. Molecules that mimic this peptide structure may also bind to the Src Homology 2 (SH2) domain and inhibit its function.

The inhibition of signal transduction using inhibitors of critical interactions has important potential utility in the development of pharmaceutical agents. A problem in this field is the identification of novel lead structures that cause such inhibition.

Several approaches have been suggested for the identification of such lead structures. One approach is determining the structure of the proteins involved and their naturally occurring binding targets or substrates. Then the relevant regions are modelled and synthetic analogues are generated. This is a difficult, expensive, time consuming and uncertain process.

A second approach is screening random naturally occurring compounds for a desired activity. This approach requires significant specialized resources. In addition, the resulting compounds may or may not be amenable to structure activity relationship studies necessary to improve activity.

Finally, linear phosphopeptides have been studied to identify those that bind to SH2 domains. The sequence specificity of those peptides may be indicative of the sequence consensus that allows for binding to the SH2 domain. However, these studies are often not particularly useful for the identification of structures that improve drug discovery. The linear peptides can adopt numerous three dimensional structures that complicate the generation of structural analogues.

In studies with phosphopeptides, the peptides contained a phosphotyrosine. The equivalent non-phosphorylated peptides bound with reduced affinity. In studies of the p85 SH2 domain, the non-phosphorylated peptides bound with greatly reduced affinity. Although Felder, S., et al., "SH2 domains exhibit high-affinity binding to tyrosine-phosphorylated peptides yet also exhibit rapid dissociation and exchange," Mol. Cell Biol., 13: 1449-1455, 1993, report that non-phosphorylated and phosphorylated Y628 (of IRS-1) may associate with the SH2 domain of p85 in a K_{d} ratio of the non-phosphorylated to the phosphorylated compound of greater than 50, most other non-phosphorylated peptides have had even greater reduced affinity. Further, no apparent binding is seen in the reported data for the interaction between non-phosphorylated Y628 and the SH2 domain in Figure 2(c) of the reference.

Previous studies have also reported peptide and non-peptide molecules that bind to SH2 domains of signal transduction proteins that are based on structures that mimic a phosphorylated peptide. See, for example, Ye, B. et al., "L-O-(2-malonyl)tyrosine: a new phosphotyrosyl mimetic for the preparation of Src homology 2 domain inhibitory peptides," J. Med. Chem., 38: 4270-4275, 1995, and Burke T.R., Jr., et al., "Nonhydrolyzable phosphotyrosyl mimetics for the preparation of phosphatase-resistant SH2 domain inhibitors," Biochemistry, 33: 6490-6494, 1994,.

Phosphopeptides are highly charged and thus are often impermeable to cells. Modifications have been made that reduce the charge of the phosphotyrosine residue. However, no study has been reported that describes a peptide having an unmodified tyrosine residue as a competitor of a natural target.

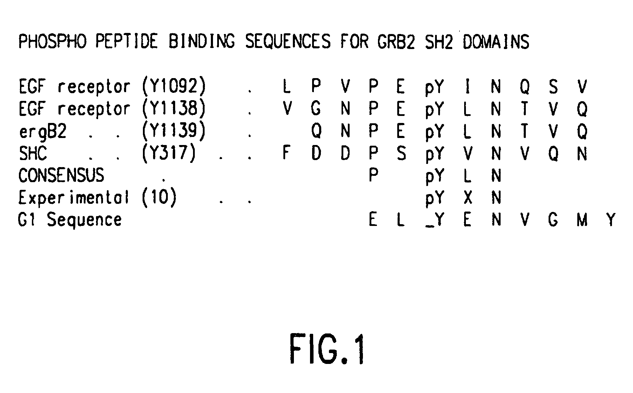

Grb2 is an example of a well established signal transduction protein in many cell control systems. Schlessinger, J. ; "SH2/SH3 signaling proteins," Curr. Opin. Genet. Dev. 4: 25-30, 1994. The natural targets for Grb2 include SHC phosphoprotein. A SHC phosphopeptide sequence that binds to the Grb2 SH2 domain, as well as other phosphopeptide sequences that bind to the Grb2 SH2 domain, are shown in Figure 1. From these sequences, a consensus sequence that binds to the Grb2 SH2 domain was determined. A consensus binding sequence was also determined experimentally by binding peptide mixtures to Grb2, as described in Songyang, Z., et al., "Specific motifs recognized by the SH2 domains of Csk, 3BP2, fps/fes, GRB-2, HCP, SHC, Syk, and Vav," Mol. Cell. Biol., 14: 2777-2785, 1994 .

### SUMMARY OF THE INVENTION

WO-A-96/23813 which has been published after the priority date claimed by the present application, discloses phosphorylated and non-phosphorylated SH2-binding peptides comprising at least the following sequence Tyr-Xaa-Asn-Xaa wherein the tyrosine is phosphorylated or not.

The present invention is directed to a non-phosphorylated peptide that binds to an SH2 domain of a signal transduction protein with high affinity. That is, the peptide binds to the SH2 domain with an affinity similar to or greater than that of at least one phosphorylated peptide that is a natural target for the protein. Because the non-phosphorylated peptide binds to an SH2 domain with an affinity similar to or greater than a natural target, it can be used to inhibit the binding of the natural target.

The present invention is also directed to a non-phosphorylated peptide that binds to the Grb2 SH2 domain with high affinity. That is, the peptide binds to the Grb2 SH2 domain with an affinity similar to or greater than that of a phosphorylated peptide derived from the SHC protein, which has a sequence DDPSpYVNVQ (SEQ ID NO: 1).

In addition, the present invention is directed to a non-phosphorylated peptide G1 comprising the following sequence: Cys-Glu-Leu-Tyr-Glu-Asn-Val -Gly-Met-Tyr-Cys (SEQ ID NO: 2), wherein the sequence has a disulfide linkage between the terminal cysteines. These flanking cysteines cause the formation of a disulfide constrained cyclic structure. The present invention is also directed to a phage containing the above sequence as a variable region.

The present invention also describes the inhibition of a signal transduction process, particularly those of the Grb2 SH2 domain, by administering a non-phosphorylated peptide.

In addition, the present invention describes a process for forming a drug to block the inhibition of a signal transduction process that uses a non-phosphorylated peptide, particularly G1, as a model.

### BRIEF DESCRIPTION OF THE DRAWINGS

Figure 1 demonstrates phosphopeptide binding sequences for Grb2 SH2 domains. Figure 1 also demonstrates a consensus sequence for the phosphopeptide binding sequences and compares these sequences to the G1 sequence.
Figure 2A is a graph demonstrating that G1 phage binds to intact Grb2.
Figure 2B is a graph demonstrating the specificity of G1 phage for Grb2.
Figure 3A is a graph demonstrating Grb2 binding to phage G1.
Figure 3B is a graph demonstrating Grb2 binding to SHC phosphopeptide.
Figure 4 is a graph demonstrating the poor binding of anti-phosphotyrosine antibodies to G1 phage.
Figure 5 is a graph demonstrating the inhibition of Grb2 binding co SHC phosphopeptide.
Figure 6 is a graph demonstrating the alteration of binding caused by the presence of various concentrations of different peptides.
Figure 7 is a graph demonstrating the requirement of an YXN sequence in the G1 peptide.
Figures 8A-C demonstrate the inhibition of complexes formed by the Grb2 SH2 domain in cellular extracts by G1TE peptide. Figure 8A demonstrates the structure of G1 and the G1TE molecule containing a thio-ether linkage. Figure 8B demonstrates the inhibition of Grb2 binding to the SHC phosphopeptide using surface plasmon resonance methods. Figure 8C demonstrates that the addition of G1TE and SHC phosphopeptide to lysates of cells inhibits the immunoprecipitation of complexes between Grb2 and p185^{erbB-2}. The binding of Grb2 to SOS1 is unaffected by G1TE peptide addition.

### DETAILED DESCRIPTION OF PREFERRED EMBODIMENTS

The present invention discloses a non-phosphorylated peptide sequence, G1, that binds to the Grb2 SH2 domain with similar affinity as compared to the SHC phosphoprotein. G1 binds to and blocks the Grb2 SH2 domain. As demonstrated in Figure 1, the G1 peptide sequence apparently conserves only one position of the primary sequence of linear phosphopeptides shown to bind preferentially to the Grb2 SH2 domain. That is, G1 contains an asparagine at position +2 to the tyrosine.

In Figure 1, the standard one letter amino acid codes have been used. The small e indicates a common but not invariant amino acid at position -1 to the phosphotyrosine (pY). In the G1 sequence, the non-phosphorylate nature of the tyrosine is indicated by a _Y. X indicates that multiple amino acids are possible at that position.

The G1 peptide demonstrates the principle that a small molecule can bind to the SH2 domain of Grb2 and inhibit its ability to bind phosphotyrosine containing proteins. Further, the G1 peptide demonstrates that non-phosphorylated peptides can bind to SH2 domains with high affinity, comparable to the binding affinity of phosphorylated peptides.

The G1 peptide can be used as a starting point for the identification of other non-phosphorylated peptides that may bind to the SH2 domain with even greater affinity. The G1 peptide can be used as a starting point for the identification of functional groups on a small molecule that are critical for binding to the SH2 domain. In addition, the G1 peptide can be used as a structural model for non-peptide molecules with SH2 domain binding activity that may be identified by screening databases of compounds for chemically similar structures. Such database screening is generally described, for example, in Sun, E. and Cohen, F.E., "Computer-assisted drug discovery--a review," Gene, 137: 127-132, 1993, and Wang, S., et al., "The discovery of novel, structurally diverse protein kinase C agonists through computer 3D-database pharmacophore search. Molecular modeling studies," J. Med. Chem., 37: 4479-4489, 1994,. Further, the G1 peptide can be used as a basis for synthesis of peptide mimetic compounds. Olson, G.L., et al., "Concepts and progress in the development of peptide mimetics," J. Med. Chem., 36: 3039-3049, 1993, teaches general principles of the process of synthesis of nonpeptide compounds that retain the biological activity of a peptide. The reference also describes some specific examples of replacement of peptide backbones.

The G1 peptide serves as a model for drugs with significant advantages. Inhibition of SH2 domain functions have previously been attempted using structures that contain a phosphotyrosine or mimic it with a similarly charged group. In contrast, the G1 peptide structure does not contain a phosphotyrosine or a similarly charged group on the tyrosine. As a result, G1 should offer improved cell penetration. Therefore, the G1 peptide sequence has significant advantages in that it and its derivatives may enter cells more efficiently than molecules based on the phosphopeptide. Gilmer, T. et al., "Peptide inhibitors of src SH3-SH2-phosphoprotein interactions," J. Biol. Chem., 269: 31711-31719, 1994, describes an unsuccessful experiment in which a phosphopeptide AcpYEEIE was used to attempt to block cell proliferation. The reference suggests that this lack of effect is likely due to poor penetration of cells and/or poor stability of the phosphotyrosine. Analogues of the G1 peptide may have the advantages of other small molecular weight pharmaceuticals including tissue penetration, bioavailability and ease of manufacture.

The present invention describes a peptide sequence that can serve as a model for peptide and/or non-peptide pharmaceuticals. In particular, the G1 peptide may be used to find other peptide or non-peptide compounds that have similar structural elements that can bind to the Grb2 SH2 domain and block its signal transduction function. These compounds could have activity as drugs, for example, for the treatment of cancer or other hyperproliferative medical conditions.

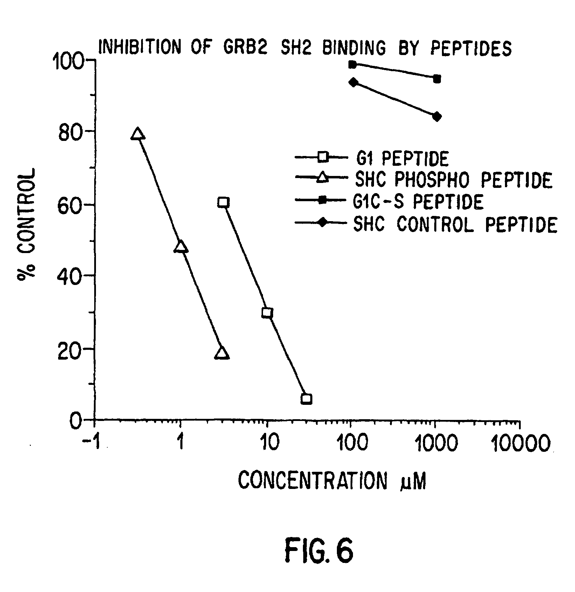

A measure of affinity of a peptide for the SH2 domain of a protein is the equilibrium dissociation constant, or K_{d}. A lower K_{d}, measured in terms of molarity, is indicative of greater affinity. Affinity of Grb2 for SHC phosphopeptide has been estimated by using surface plasmon resonance. Pelicci, G., et al., "The motogenic and mitogenic responses to HGF are amplified by the Shc adaptor protein," Oncogene, 10: 1631-1638, 1995. Binding of SH2 domains of Grb2 to SHC phosphopeptide bound to a surface suggests a K_{d} of 15 nM. In experiments in which the affinity of SHC phosphopeptide and G1 for GST-Grb2 in solution are directly compared, it was determined that the affinities are about 10 fold different, as demonstrated in Figure 6 discussed below.

A peptide having affinity for the SH2 domain can be used to inhibit the binding of a natural target of the SH2 domain. IC₅₀ measures the concentration at which the peptides cause 50% inhibition of the natural target. A lower IC₅₀, measured in terms of molarity, is indicative of greater inhibition and thus greater affinity.

The present invention is directed to a non-phosphorylaced peptide that binds to an SH2 domain of a signal transduction protein with high affinity. That is, the peptide or composition of the present invention binds to the SH2 domain with an affinity similar to or greater than that of at least one phosphorylated peptide that is a natural target for the protein.

The following embodiment is useful for understanding the invention. The peptide or composition of the present invention binds to the SH2 domain with an affinity for the SH2 domain greater than or within fifty fold less than the affinity of a phosphorylated natural target. Preferably, the affinity of the peptide or composition is greater than or within twenty-five fold less, and more preferably greater than or within fifteen fold less, than the affinity of a natural target. Thus, the peptide or composition has a K_{d} and/or an IC₅₀ for an SH₂ domain less than or within fifty fold greater and preferably within twenty-five fold greater, and more preferably within fifteen fold greater, than that of at least one natural target of the SH₂ domain.

As used herein, the phrase "non-phosphorylated peptide" refers to a peptide that contains a tyrosine residue that has not been modified by a phosphate or a similarly charged group, such as a phosphorate or a malonate. In terms of the present description, by "similarly charged group" is meant a group having a negative charge at normal intracellular pH and/or a group that is highly polar. Highly polar group may include groups that are at least as or more polar than oxygen. Highly polar groups may also include groups that are more polar than nitrogen. In another embodiment of the present invention, the "non-phosphorylated peptide" contains an unmodified tyrosine.

As used herein, the phrase "natural target" refers to phosphopeptides that are naturally occurring in vivo and bind with the SH2 domain of a signal transduction protein with sufficient affinity such that a signal is produced. In a preferred embodiment, the natural target is the natural target having the greatest affinity for the SH2 domain of a particular signal transduction protein.

The present invention is also directed to a non-phosphorylated peptide that binds to the Grb2 SH2 domain with high affinity, that is, a peptide that binds to the Grb2 SH2 domain with an affinity similar to that of a phosphorylated peptide derived from the SHC protein having a sequence DDPSpYVNVQ (SEQ ID NO: 1). This sequence of the SHC protein is a known target for the Grb2 SH2 domain. Rozakis-Adcock, et al., "Association of the Shc and Grb2/Sem5 SH2-containing proteins is implicated in activation of the Ras pathway by tyrosine kinases," Nature, 360: 689-692, 1992,

The following embodiment is useful for understanding the invention. The affinity of the peptide for the Grb2 SH2 domain is greater than or within fifty fold less than the affinity of the SHC protein having SEQ ID NO: 1. Thus, the peptide has a K_{d} and/or an IC₅₀ for an Grb2 SH₂ domain less than or within fifty fold greater and preferably within twenty-five fold greater, and more preferably within fifteen fold greater, than that of the SHC protein.

The present invention is also directed to a non-phosphorylated peptide comprising sequence G1 that binds to Grb2 SH2 domain with similar affinity. Direct cyclic peptides in which the terminal cysteines are replaced by a peptide bond or with an intervening amino acid are possible.

G1 may be used alone or as part of a biologically entity or a synthetic structure, which may include a phage. The insertion of a sequence into a phage is within the knowledge of one of ordinary skill in the art. For example, G1 may be inserted into a fd phage, such as fUSES. The composition may also be part of a fusion protein, such as with Gst.

The present invention is also directed to: the inhibition of a signal transduction process, particularly those of the Grb2 SH2 domain, by administering a non-phosphorylated peptide as discussed above. The peptide of the present invention may be administered by any known method, including oral and parenteral administration. In addition, the peptide may be administered alone or with a pharmaceutically acceptable carrier. Dosages may be routinely determined and varied to obtain the desired effect.

In addition, the present invention may be applied to a process for forming a drug to block the inhibition of a signal transduction process that uses a non-phosphorylated peptide, particularly G1, as a model. That is, the present invention is directed to processes in which G1 is used as a basis for creating new drugs that block the inhibition of a signal transduction process. Methods for the conversion of a peptide into a non-peptide mimetic compound include: use of non L-amino acids or other amino acid analogues in synthesis, determination of the structure of a peptide and use of this structure to guide synthesis of similar non-peptide molecules, and use of the special positioning of essential chemical groups in a peptide to search databases for compounds of similar structure.

### IDENTIFICATION OF G1 PEPTIDE SEQUENCE

The G1 peptide sequence was identified using phage display techniques similar to those taught in U.S. Patent No. 5,223,409, to Ladner et al., which is incorporated herein in its entirety by reference. A library was constructed to contain more than 10⁷ different peptide sequences bounded by cysteine residues inserted into the Gene III protein of the fd phage fUSES. In order to identify the G1 peptide, a variation of standard methodology for purifying specific phages bound to a target protein from among the large diversity of the library was used. Scott, J.K. and Smith, G.P., "Searching for peptide ligands with an epitope library," Science, 249: 386-390, 1990.

The Grb2 protein was generated as a glutathione S-transferase (GST) fusion protein using recombinant expression vectors in E. coli as taught in Sastry, et al., "Quantitative analysis of Grb2-Sos1 interactions: the N-terminal SH3 domain Grb2 mediates affinity," Oncogene, 11:1107-12, 1995. The phage library was allowed to bind to the recombinant Grb2 protein. The GST-Grb2 proteins were collected on glutathione sepharose. Unbound phages were removed by washing and bound phages were eluted. These Grb2 binding phages where allowed to infect E. coli and the amplified phages were isolated. Phage binding to GST bound to glutathione sepharose were removed. This affinity isolation binding to Grb2 was repeated twice.

Following the third Grb2 binding step, eighteen resulting phages were isolated. The isolated phages were subjected to nucleotide sequencing. The variable region of each phage showed an identical nucleotide sequence and predicted protein sequence. The predicted sequence is that of G1. Variations on the above phage selection procedure may yield other phages either related to the G1 peptide or of a different sequence class.

In order to determine which region of the Grb2 protein is bound by the G1 peptide, ELISA type binding assays were conducted as described in Harlow, E. and Lane, D., Antibodies: A Laboratory Manual, Cold Spring Harbor Laboratories, 1988,. In the experiment, four different recombinant proteins were bound to polystyrene 96 well microtiter plates at 10 µg/well in 1X PBS for 16 hours at 4°C. These recombinant proteins were intact GST-Grb2 fusion protein, the N-terminal Grb2-SH3 domain, the C-terminal Grb2-SH3 domain and GST alone. The plates were then blocked with Casein Block (Pierce), 10mM Tris pH 7.5, and 150mM NaCl (TBS). The plates were washed three times with wash buffer containing 0.1% Tween (TBS).

G1 phage extracts were prepared by inoculating a culture containing 40 µg/ml tetracycline with an active tetracycline resistant phage containing bacterial colony and growing overnight. Phage stocks were allowed to bind to the immobilized target proteins for two hours at room temperature. The plates were then washed twice with wash buffer. Sheep anti-phage antibody was incubated in a blocking buffer at 1:1000 dilution for one hour at room temperature. Donkey anti-sheep antibody coupled to alkaline phosphatase was incubated with the plates (1:1000 dilution) for one hour at room temperature. The plates were washed twice with wash buffer. Then the color was developed using a p-nitro-phenyl phosphate (PNP) substrate.

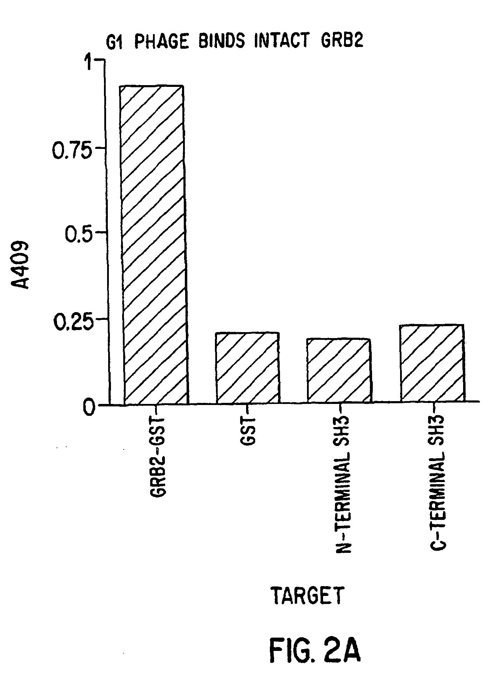

As seen in Figure 2A, only the full length GST-Grb2 proteins bound to the phage. This indicates that the G1 phage binds to the SH2 domain and not to the SH3 domains or the GST portion of the recombinant proteins.

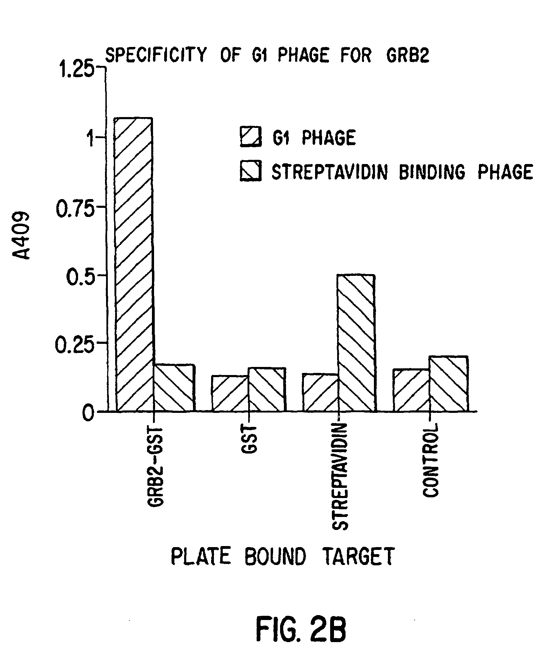

A comparative experiment was also conducted using the same procedures as described above, except that an fd control phage, which was identical to the G1 phage except that it contained a different peptide sequence not found in G1 and is specific for streptavidin binding, was used instead of G1 phage. Figure 2B demonstrates the binding of the control phage as compared to the G1 phage. The binding pattern indicates that binding to the Grb2 SH2 domain requires the G1 peptide sequence and is not a general characteristic of a fd phage.

In order to determine whether the G1 phage binds with high affinity to the Grb2 SH2 domain, the concentration dependence of binding of GST-Grb2 to immobilized phage targets was measured. The concentration of GST-Grb2 was varied in a binding reaction and the extent of bound GST-Grb2 was measured using anti-GST antibodies. The binding of GST-Grb2 to G1 phage was compared with the binding of GST-Grb2 to a phosphorylated peptide derived from the SHC protein (sequence: DDPSpYVNVO), a known target of Grb2 SH2 domain, *in vivo*.

In the experiment, target protein G1 phage or SHC phosphopeptide was bound to polystyrene 96 well microtiter plates at 1 µg/well in PBS buffer for 16 hours at 4°C. The G₁ phage was prepared by PEG precipitation of stationary phage bacterial cultures of G1 infected E. coli. The SHC phosphopeptide target was prepared by crosslinking the phosphopeptide to Bovine Serum Albumin (BSA) by a 1-ethyl-3-(3-dimethylaminopropyl) carbodiimide hydrochloride -(EDC) using standard procedures, as taught in Harlow, E. and Lane, D., Antibodies: A Laboratory manual, Cold Spring Harbor Laboratories, 1988,. The plates were then blocked with blocking buffer containing 5% BSA, 10mM Tris pH 7.5, 150mM NaCl (TBS) and 1mM NaVO₄. The plates were washed three times with washing buffer containing 0.1% BSA in TBS.

The GST-Grb2 protein was prepared as described in Sastry, et al., "Quantitative analysis of Grb2-Sos1 interactions: the N-terminal SH3 domain Grb2 mediates affinity," Oncogene, 11:1107-12, 1995. GST-Grb2 was incubated in the plate wells for two hours at room temperature. Non-specific binding reactions were inhibited using 5% BSA. Phosphatases were inhibited by 1 mM NAVO₄ in the binding buffers. The plates were washed twice with wash buffer.

Binding was detected using sequential reactions of anti-GST antibody antisera (Santa Cruz Biochem) followed by goat anti-mouse IgG antibody coupled to alkaline phosphatase followed by calorimetric development using PNP substrate. In particular, anti-GST monoclonal antibody was incubated in blocking buffer at 1ng/ml for one hour at room temperature. Then goat anti-mouse IgG antibody antisera coupled to alkaline phosphatase was incubated with the plates (1:1000 dilution) for one hour at room temperature. The plates were washed twice with wash buffer. Then the color was developed using a PNP substrate.

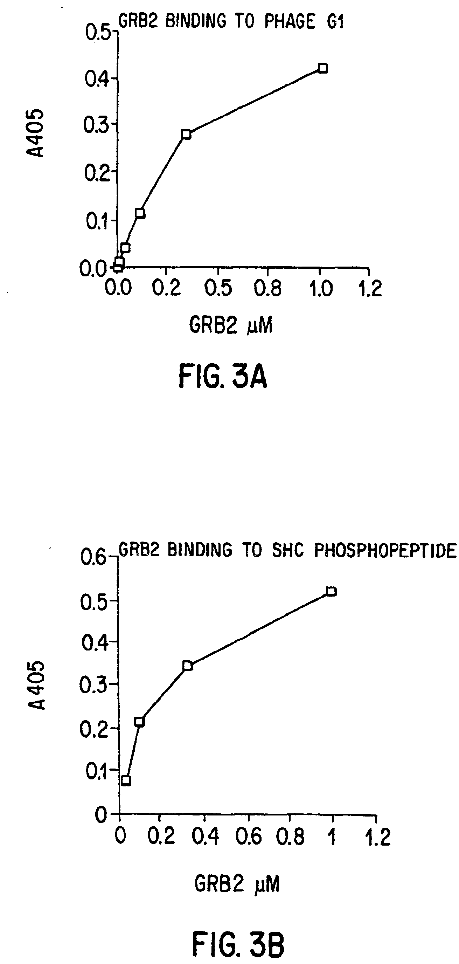

As shown in Figures 3A and 3B, there is very similar binding of Grb2 to the G1 phage as to the SHC phosphopeptide target. Zero absorbance at an optical density of 409 nm (A409) represents background with no added Grb2.

Bacteria do not contain enzymes that phosphorylate tyrosine residues. However, in order to confirm that the G1 phage peptide does not become phosphorylated during phage propagation in E. coli, the reactivity of the G1 phage towards anti-phosphotyrosine antibodies was tested in assays where the G1 phage and SHC phosphopeptide were bound to plates as above.

In the experiment, target protein G1 phage or SHC phosphopeptide was bound to polystyrene 96 well microtiter plates at 1 µg/well in PBS buffer for 16 hours at 4°C. The G1 phage was prepared by PEG precipitation of stationary phage bacterial cultures of G1 infected E. coli. The SHC phosphopeptide target was prepared by EDC crosslinking to BSA as described above. The plates were then blocked with blocking buffer containing 5% BSA, 10mM Tris pH 7.5, 150mM NaCl (TBS) and 1mM NaVO₄. The plates were then washed three times with wash buffer containing 0.1% BSA in TBS.

The GST-Grb2 protein was prepared as previously described above. GST-Grb2 (0.5*µ*M) was incubated in plate wells for two hours at room temperature. Monoclonal anti-phosphotyrosine antibody (Santa Cruz Biochemicals) was incubated with the plates at 1ng/ml for two hours at room temperature. The plates were washed twice with wash buffer. Anti-GST monoclonal antibody (Santa Cruz Biochem) was incubated in blocking buffer at lng/ml for one hour at room temperature. Goat anti-mouse antibody coupled to alkaline phosphatase was incubated with the plates (1:1000 dilution) for one hour at room temperature. The plates were washed twice with wash buffer and color was developed using a PNP substrate.

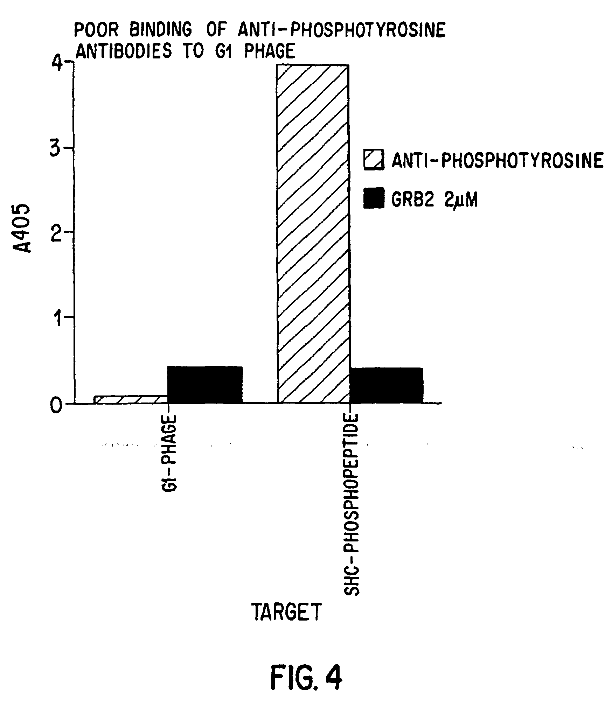

As indicated in Figure 4, there is no reactivity with the G1 phage while the positive control SHC phosphopeptide gives a strong signal. This indicates that the G1 phage peptide does not contain a phosphotyrosine residue, i.e., the G1 phage peptide does not become phosphorylated during phage propagation.

In order to show that the G1 phage and SHC phosphopeptide compete for the same binding site on Grb2, it was investigated whether the SHC phosphopeptide can block the binding of Grb2 to immobilized G1 phage. In the experiment, target protein G1 phage or SHC phosphopeptide was bound to polystyrene 96 well microtiter plates at 1 µg/well in PBS buffer for 16 hours at 4°C. The G1 phage was prepared by PEG precipitation of stationary phage bacterial cultures of G1 infected E. coli. The SHC phosphopeptide target was prepared by EDC crosslinking to BSA using standard procedure as described above. The plates were then blocked with blocking buffer containing 5% BSA, 10mM Tris pH 7.5, 150mM NaCl (TBS) and 1mM NaVO₄. The plates were then washed three times with wash buffer containing 0.1% BSA in TBS.

The GST-Grb2 protein was prepared as described above. GST-Grb2, at a concentration of 0.5*µ*M, was preincubated in binding buffer with various concentrations of soluble SHC phosphopeptide for two hours and allowed to bind to the plate bound targets. The plates were washed twice with wash buffer. Anti-GST monoclonal antibody (Santa Cruz Biochem) was incubated in blocking buffer at 1ng/ml for 1 hour at room temperature. Goat anti-mouse antibody coupled to alkaline phosphatase was incubated with the plates (1:1000 dilution) for one hour at room temperature. The plates were washed twice with wash buffer and color was developed using a PNP substrate.

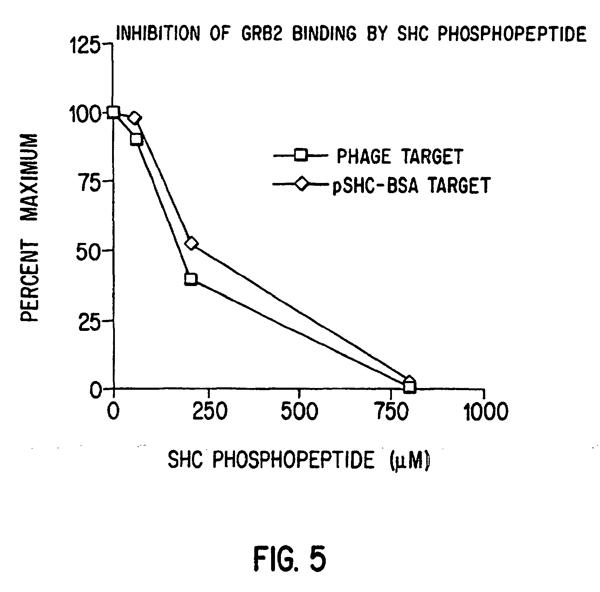

As shown in Figure 5, the concentrations of SHC-phosphopeptide required to block binding to G1 phage and to the homologous target are nearly identical. This indicates that the G1 phage peptide binds to either the same site as the SHC phosphopeptide or very close thereto. Zero A409 represents background with no added Grb2.

In order to gain insight into the binding affinity of the G1 peptide outside of the context of the phage, Gene III protein, the peptide was chemically synthesized and a form containing the cysteine residues in oxidized, disulfide bonded form was isolated. Standard methods were used for solid phase chemical synthesis of the peptide. The oxidized, disulfide bonded peptide formed spontaneously during the isolation of the peptide. The formation of this conformation was confirmed by mass spectrometric analysis, indicating a molecular weight of 1321. In addition, 10 mM DTT induced alteration in mobility in C18 chromatography experiments using an acetonitrile gradient in 0.5% trifluoroacetic acid. These experiments demonstrate an altered conformation on reduction and therefore confirm the presence of the disulfide bond.

The G1 peptide (CELYENVGMYC [disulfide bonded]) was used as an inhibitor of the interaction of the Grb2 with a SHC phosphopeptide in surface plasmon resonance (Biacore) experiments. The SHC phosphopeptide was attached to the solid surface by incorporation of a biotin at the N-terminus as described in Pelicci, G., et al., "The motogenic and mitogenic responses to HGF are amplified by the Shc adaptor protein," Oncogene, 10: 1631-1638, 1995. This biotinylated phospho-SHC peptide was then bound to the streptavidin on the surface of SA5 chips (Pharmacia-Biacore). The binding was conducted at a 2nM and 5 *µ*l/minute for 10 minutes. The peptides were mixed with 200 nM GST-Grb2 in HBS buffer (Pharmacia Biacore) and allowed to bind for 10 minutes at a flow rate of 5 *µ*l/minute. The Ru change at equilibrium was calculated and the percent of control (no added peptide) binding was calculated.

The G1 peptide was compared to SHC phosphopeptide (DDPSpYVNVQ), G1C-S (SELYENVGMYS), which is similar to G1 but wherein the cysteines are replaced with serines, and SHC control peptide (DDPSYVNVQ), in which the tyrosine is not phosphorated. The peptides were used at the concentrations indicated in Figure 6.

The results are shown in Figure 6. The G1C-S peptide demonstrates that the disulfide bond may be required. In particular, the conformational constraint imparted by the disulfide bond in the G1 peptide may be required. The SHC control demonstrates the poor affinity of the linear non-phosphorylated SHC peptide.

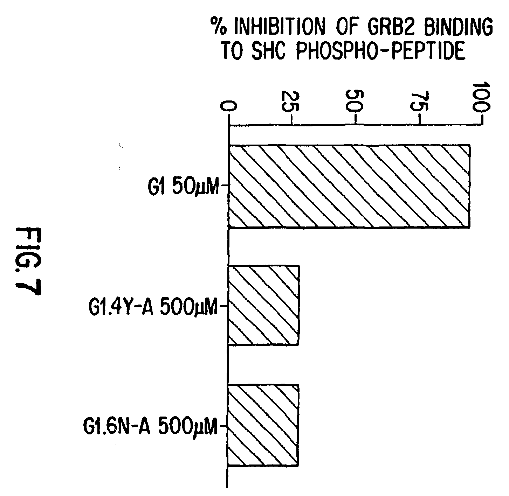

The alignment of the G1 sequence, shown in Figure 1, with Grb2 phosphopeptide ligand suggests the importance of tyrosyl and asparagine residues at positions 4 and 6, respectively. These residues have also previously been shown to be required for high affinity binding of pTyr containing peptides to Grb2 SH2 domain. Songyang et al. Mol. Cell Biol., 14:2777-2785 (1994). The requirement of these amino acids in the G1 peptide sequence was tested by synthesizing peptides with alanine substitutions at these positions. G1.4Y-A contains an alanine substitution in place of the tyrosine at position 4 (CELAENVGMYC) and G16N-A contains an alanine substitution for the asparagine at position 6 (CELYEAVGMYC). No inhibition of Grb2 binding to the SHC phosphopeptide was seen with either peptide at a concentration of 50 *µ*M. The binding of peptide to the Grb2 SH2 domain was measured by surface plasmon resonance (Pharmacia Biacore). The percent inhibition was calculated by comparison of the Ru_{eq} in the presence of peptide with the Ru_{eq} with no inhibiting peptide present. As shown in Figure 7, replacement of the tyrosine or asparagine residues of G1 greatly diminish its ability to bind to Grb2 and block association with SHC phosphopeptide. No inhibiting activity was seen at 50 µM and limited activity at 500 µM. These results strongly suggest that G1 binds the ligand binding region of the Grb2 SH2 domain using some of the same amino acid contacts as pTyr containing peptides.

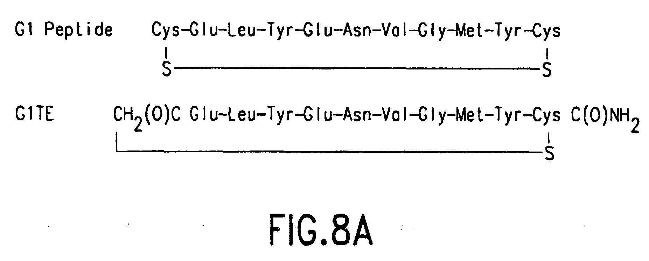

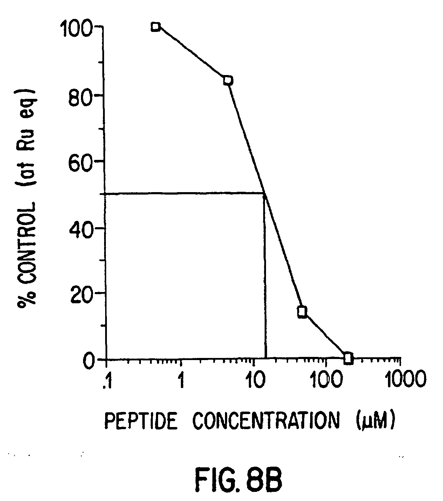

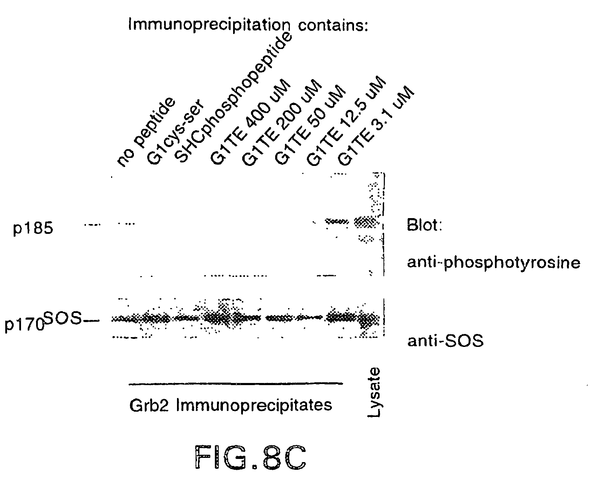

In order to verify that the interaction between G1 and Grb2 is sufficient to inhibit the Grb2 SH2 domain association with intact phosphoproteins, experiments in cell extracts were conducted. Cell lysates derived from the breast cancer cell line MDA-MB-453 which contains an amplified *erbB-2* gene and overexpressed p185^{erbB-2} were used. In the cell lines, an overexpressed p185^{erbB-2} generates abundant autophosphorylation. Due to the concern that the G1 peptide requires a disulfide bond and reduction might occur in the cell lysate conditions, a similar molecule, G1TE, was synthesized in which the disulfide structure has been replaced by a thio-ether bond. Binding of G1TE to GST-Grb2 and inhibition of SH2 function *in vitro* demonstrate similar IC₅₀ values compared to G1 (Figure 8A-C). When added to cell lysates, the G1TE molecule is able to diminish the amount of p185 pTyr containing protein that co-immunoprecipitate with Grb2. SHC phosphopeptide also showed similar ability. SH2 domains associate with phosphopeptides with high affinity but display very fast dissociation and association kinetics. Thus, a "preformed" complex of Grb2 bound to p185^{erbB-2} can be sensitive to peptides that prevent the reassociation component of the equilibrium. The relative effectiveness of G1TE is these assays is comparable to that seen in the SPR analysis with partial interference at 50 *µ*M (Figure 8A-C). No effect of peptides was seen on binding of Grb2 to SOS1, an SH3 domain mediated interaction. These results indicate that the association of Grb2 with p185^{erbB-2} can be prevented by small non-phosphorylated peptides like G1TE.

The results described in Figures 1-8 indicate that the non-phosphorylated G1 phage contains a peptide that binds with high affinity to the phosphopeptide binding site on the SH2 domain of Grb2. As a result, the present invention demonstrates that peptides of constrained structure can have novel and unexpected activity on targets involved in signal transduction.

Similar results could be obtained by screening other types of combinatorial libraries displaying cyclic peptides. Peptide combinatorial libraries have been reported where the peptide are in solution or bound to beads. See, for example, Burke, T.R., Jr., et al., "Nonhydrolyzable phosphotyrosyl mimetics for the preparation of phosphatase-resistant SH2 domain inhibitors," Biochemistry, 33: 6490-6494, 1994, and Gilmer, T., et al., "Peptide inhibitors of src SH3-SH2-phosphoprotein interactions," J. Biol. Chem., 269: 31711-31719, 1994.

The cyclic peptides used in the above examples were formed by disulfide or thio-ether bonds but could be formed by other chemical linkages. Direct cyclization of peptide sequences using a peptide bond in place of the two cysteine residuals or with intervening amino acid is possible. Peptides of constrained structure could also contain cysteines separated by 4-12 amino acids. Peptides could also contain non-natural amino acids such as D amino acids. Modifications in peptide bonds and other peptide mimetic structures are possible. Furthermore, not all of the amino acids of the reported sequence may be necessary for activity. Peptides with single or multiple conservative substitutions are likely to be active. Peptides where non-conservative alterations do not change the overall three dimensional conformation of the peptide are likely to be active in a manner similar to the G1 peptide. The G1 peptide is likely to contain 2-8 functional groups that interact with the Grb2 SH2 domain. The three dimensional position of these groups may be mimicked by synthetic or natural products. Such compounds may also have activity as SH2 antagonists, and are encompassed by the present invention.

The present invention thus demonstrates that SH2 domains can be inhibited by non-phosphorylated structures in which the tyrosine has not been modified by a phospho or a similarly charged group. Therefore other SH2 domain interactions of other proteins, such as SHC, crk, nck, ragGAP, P13kinase, PLC gamma, src, or others, may also have non-phosphorylated inhibitors. This is in contrast to efforts to mimic the phosphotyrosine with other modifications or blocking groups.

## Claims

1. A cyclic peptide comprising the amino acid sequence of Glu-Leu-Xaa-Glu-Asn-Val-Gly-Met-Tyr
wherein Xaa is an unphosphorylated tyrosine and wherein the peptide does not comprise a phosphotyrosine or a similarly charged group on the tyrosine.

2. The peptide of claim 1, wherein the similarly charged group is selected from the group consisting of phosphorate and malonate.

3. A cyclic peptide consisting of the amino acid sequence of Glu-Leu-Xaa-Glu-Asn-Val-Gly-Met-Tyr
wherein Xaa is an unphosphorylated tyrosine.

4. The peptide of any one of the preceding claims, wherein the peptide is cyclized by a bond selected from the group consisting of a disulfide bond, a thio-ether bond and a peptide bond.

5. The peptide of any one of the preceding claims, wherein the terminal residues of the peptide are linked together.

6. The peptide of claim 5, wherein the terminal residues are cysteine residues.

7. A phage containing the nucleic acid sequence encoding the peptide of claims 1 to 6.

## Revendications

1. Peptide cyclique comprenant la séquence d'acides aminés de Glu-Leu-Xaa-Glu-Asn-Val-Gly-Met-Tyr
dans laquelle Xaa est une tyrosine non phosphorylée et dans laquelle le peptide ne comprend pas de phosphotyrosine ni de groupe de charge similaire sur la tyrosine.

2. Peptide selon la revendication 1, dans lequel le groupe chargé de manière similaire est choisi dans le groupe comprenant le phosphorate et le malonate.

3. Peptide cyclique consistant en la séquence d'acides aminés de Glu-Leu-Xaa-Glu-Asn-Val-Gly-Met-Tyr
dans laquelle Xaa est une tyrosine non phosphorylée.

4. Peptide selon l'une quelconque des revendications précédentes, dans lequel le peptide est cyclisé par une liaison choisie dans le groupe comprenant un pont disulfure, une liaison thio-éther et une liaison peptidique.

5. Peptide selon l'une quelconque des revendications précédentes, dans lequel les résidus terminaux du peptide sont liés ensemble.

6. Peptide selon la revendication 5, dans lequel les résidus terminaux sont des résidus cystéine.

7. Phage contenant la séquence d'acide nucléique codant le peptide des revendications 1 à 6.

## Patentansprüche

1. Zyklisches Peptid, umfassend die Aminosäuresequenz Glu-Leu-Xaa-Glu-Asn-Val-Gly-Met-Tyr,
wobei Xaa für ein nicht-phosphoryliertes Tyrosin steht und wobei das Peptid nicht ein Phosphotyrosin oder eine ähnlich geladene Gruppe an dem Tyrosin umfaßt.

2. Peptid nach Anspruch 1, wobei die ähnlich geladene Gruppe ausgewählt ist aus der Gruppe bestehend aus phosphorat und Malonat.

3. Zyklisches Peptid, bestehend aus der Aminosäuresequenz Glu-Leu-Xaa-Glu-Asn-val-Gly-Met-Tyr,
wobei Xaa für ein nicht-phosphoryliertes Tyrosin steht.

4. Peptid nach einem der vorhergehenden Ansprüche, wobei das Peptid über eine Bindung, ausgewählt aus der Gruppe bestehend aus einer Disulfidbindung, einer Thioetherbindung und einer Peptidbindung, zyklisiert ist.

5. Peptid nach einem der vorhergehenden Ansprüche, wobei die endständigen Reste des Peptids miteinander veknüpft sind.

6. Peptid nach Anspruch 5, wobei es sich bei den endständigen Resten um Cysteinreste handelt.

7. Phage, enthaltend die das Peptid nach den Ansprüchen 1 bis 6 codierende Nukleinsäuresequenz.
